(19) **Europäisches Patentamt European Patent Office Office européen des brevets**

(11) **EP 4 661 028 A1**

(12) **EUROPEAN PATENT APPLICATION**

(43) Date of publication:
10.12.2025 Bulletin 2025/50

(21) Application number: 25195232.1

(22) Date of filing: 21.08.2024

(51) International Patent Classification (IPC):
*G16H 50/70* (2018.01)

(52) Cooperative Patent Classification (CPC):
A61B 5/02405; A61B 5/0245; A61B 5/352;
A61B 5/361; A61B 5/7267; A61B 5/7275;
G06N 3/044; G06N 3/045; G06N 3/0455;
G06N 3/0464; G06N 3/047; G06N 3/0475;
G06N 3/048; G06N 3/08; G06N 3/084; (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 22.08.2023 KR 20230110112
06.08.2024 KR 20240104439

(62) Document number(s) of the earlier application(s) in accordance with Art. 76 EPC:
**24195723.2 / 4 512 322**

(71) Applicant: **Synergy A.I. Co. Ltd.**
**Seoul (KR)**

(72) Inventors:
• **PARK, Junbeom**
**Seoul (KR)**
• **SHIN, Taeyoung**
**Seoul (KR)**
• **KIM, Yehyun**
**Seoul (KR)**
• **LEE, Myeonggyu**
**Seoul (KR)**
• **KIM, Jihoon**
**Daegu (KR)**

(74) Representative: **Aldridge, Henry Alexander et al**
**Venner Shipley LLP**
**200 Aldersgate**
**London EC1A 4HD (GB)**

Remarks:
This application was filed on 11.08.2025 as a divisional application to the application mentioned under INID code 62.

(54) **METHOD, SERVER, AND COMPUTER PROGRAM FOR GENERATING HEART DISEASES PREDICTION MODEL**

(57) Proposed is a method of generating a model for predicting heart disease according to various exemplary embodiments of the present disclosure. The method includes obtaining a plurality of electrocardiogram data and generating the model for predicting heart disease on the basis of the plurality of electrocardiogram data. The generating of the model includes constructing a learning data set on the basis of the electrocardiogram data, and generating a model for predicting a risk of heart disease by performing training on one or more network functions on the basis of the learning data set, wherein the learning data set includes a first learning data set and a second learning data set which are classified for different training purposes, and the model for predicting the risk of heart disease stratifies and provides prediction information on the risk of future heart disease on the basis of a patient's electrocardiogram data.

**EP 4 661 028 A1**

(52) Cooperative Patent Classification (CPC): (Cont.)
      **G06N 3/088; G06N 3/09; G06N 3/094; G06N 5/01;**
      **G06N 7/01; G06N 20/00; G06N 20/10;**
      **G06N 20/20; G16H 50/20; G16H 50/30;**
      **G16H 50/50; G16H 50/70**

**Description**

CROSS REFERENCE TO RELATED APPLICATION

The present application claims priority to Korean Patent Application No. 10-2023-0110112, filed on August 22, 2023, and Korean Patent Application No. 10-2024-0104439, filed on August 06, 2024, the entire contents of which are incorporated herein for all purposes by this reference.

BACKGROUND

Technical Field

Various exemplary embodiments of the present disclosure relate to a method for providing a neural network model that predicts whether heart disease may occur in a patient in the future and, more specifically, to a technology of evaluating and predicting the risk of arrhythmia including atrial fibrillation and other heart diseases on the basis of electrocardiogram data by utilizing a deep learning model.

Description of the Related Art

Electrocardiogram (ECG) data has been widely used as an important tool for diagnosing heart conditions by recording the electrical activity of the heart. Conventional multi-lead electrocardiographs have played a key role in diagnosing arrhythmias, especially heart diseases such as atrial fibrillation by detecting abnormalities in the heart's rhythm and electrical conduction. Arrhythmias, including atrial fibrillation, are common diseases characterized by irregular or abnormal heartbeats and are often asymptomatic or have mild symptoms so that it is difficult to detect without proper examination. When left untreated, such arrhythmia can lead to serious health complications such as stroke, cardiac failure, and sudden cardiac death and, particularly, atrial fibrillation is one of the important types of arrhythmia because it is associated with an increased risk of stroke and cardiac failure. Therefore, it is very important to early detect and intervene atrial fibrillation and arrhythmia for effectively managing these diseases.

Meanwhile, existing electrocardiograms are mainly focused on diagnosis, and there are limitations in predicting or stratifying the risk of future heart disease. This is because existing electrocardiogram analysis methods mainly rely on statistical analysis. Statistical methodologies do not sufficiently reflect nonlinear and complex patterns, and have difficulty in precisely evaluating the heart condition of each patient and the risk of various arrhythmias.

Moreover, statistical-based models have poor performance in comparison with deep-learning-based probabilistic models in processing large amounts of data. Electrocardiogram data may be affected by signal overlaps such as muscle movement, respiration, and electrical noise from other equipment. These external factors can degrade the accuracy of statistical methodologies, resulting in a significant obstacle to accurately stratifying and quantifying the risk of heart disease.

Documents of Related Art

(Patent Document 1) Korean Patent Application Publication No. 10-2022-0104583

SUMMARY

A task to be solved by the present disclosure is devised in response to the background technology described before, and is to provide a technology capable of predicting and stratifying the risk of future heart disease on the basis of electrocardiogram data.

Tasks to be solved by the present disclosure are not limited to the tasks mentioned above, and other tasks not mentioned will be clearly understood by those skilled in the art from the following description.

A method of generating a model for predicting heart disease is disclosed according to an exemplary embodiment of the present disclosure in order to solve the described tasks. The method includes obtaining a plurality of electrocardiogram data and generating the model for predicting heart disease on the basis of the plurality of electrocardiogram data.

In an alternative exemplary embodiment, the generating of the model for predicting heart disease may include constructing a learning data set on the basis of the plurality of electrocardiogram data and generating a model for predicting a risk of heart disease by performing training on one or more network functions on the basis of the learning data set, wherein the learning data set may include a first learning data set and a second learning data set which are classified for different training purposes and wherein the model for predicting the risk of heart disease may stratify and provide prediction information on the risk of future heart disease on the basis of a patient's electrocardiogram data.

In an alternative exemplary embodiment, the first learning data set may include data for training corresponding to a process of transforming features of electrocardiogram data into a feature space, and the second learning data set may include data for a calibration related to a heart risk prediction.

In an alternative exemplary embodiment, the constructing of the learning data set may include performing preprocessing on the plurality of electrocardiogram data, and the performing the preprocessing may include performing a noisy preprocessing on the plurality of electrocardiogram data, generating a plurality of ROI signals by segmenting the plurality of noisy preprocessed electrocardiogram data into a predefined window size, and extracting an HRV feature information in a lead unit of the plurality of electrocardiogram data, wherein the HRV feature information includes indicative information regarding a variability between heart rate intervals.

In an alternative exemplary embodiment, the generating of the model for predicting the risk of heart disease may include generating an embedding model through a masking-based self-supervised learning by utilizing the first learning data set, performing an embedding which extracts a latent vector corresponding to each of the plurality of ROI signals corresponding to the second learning data set by utilizing the embedding model, performing clustering on the latent vectors by utilizing a clustering model, and constructing a vector database (DB) on the basis of the latent vectors and information on each cluster corresponding to each latent vector.

In an alternative exemplary embodiment, the generating of the embedding model may include deriving self-supervised learning so that the self-reconstruction model generates an output similar to the inputted data by processing data included in the first learning data set as an input into the self-reconstruction model, and generating the embedding model by extracting an encoder from the self-reconstruction model where the training is completed, wherein the self-reconstruction model, which is a neural network model that masks a part of the inputted data and restores the masked part, includes a dimension reduction network function and a dimension restoration network function.

In an alternative exemplary embodiment, the clustering model may perform the clustering based on a similarity distance between latent vectors corresponding to each of the plurality of ROI signals, and the performing of the clustering may include extracting a representative vector for each of a plurality of clusters corresponding to a clustering result.

In an alternative exemplary embodiment, the method may include obtaining electrocardiogram data of a subject to be predicted, performing the preprocessing on electrocardiogram data of the subject to be predicted, generating a plurality of latent vectors corresponding to the completely preprocessed electrocardiogram data by utilizing the embedding model, classifying each of the plurality of latent vectors into one of the plurality of clusters by comparing each of the plurality of latent vectors with each representative vector corresponding to each of the plurality of clusters, and generating a stratified information regarding the risk of heart disease on the basis of a classification result where each of the plurality of latent vectors is classified into the plurality of clusters.

In an alternative exemplary embodiment, the generating of the model for predicting the risk of heart disease may include obtaining user meta information corresponding to each of the plurality of electrocardiogram data, obtaining the plurality of latent vectors from the vector database and a cluster information corresponding to the plurality of latent vectors, and generating the model for predicting the risk of heart disease by performing the training on a plurality of tree models and by performing ensemble learning that integrates an output of each tree model on the basis of the HRV feature information, the user meta information, the plurality of latent vectors, and the cluster information corresponding to the latent vector.

In an alternative exemplary embodiment, the generating of the model for predicting heart disease may further include classifying the plurality of obtained electrocardiogram data into a training data set, a validation data set, and a test data set, obtaining ROI electrocardiogram data segmented into a predefined window size from each of electrocardiogram data classified into the training data set, the validation data set, and the test data set, training a deep learning model to predict a heart disease class of each first ROI electrocardiogram data in response to inputting the first ROI electrocardiogram data obtained from electrocardiogram data included in the training data set into the deep learning model for predicting the patient's heart disease, and determining a threshold value for distinguishing the heart disease class by applying second ROI electrocardiogram data obtained from electrocardiogram data included the validation data set to the trained deep learning model.

In an alternative exemplary embodiment, the determining may include collecting a probability score for each of ROI electrocardiogram data separated from the same electrocardiogram data for each of the second ROI electrocardiogram data, deriving a final probability score by averaging the probability score for each of the collected second ROI electrocardiogram data, and fine-tuning the threshold value for distinguishing the heart disease class on the basis of the derived final probability score.

In an alternative exemplary embodiment, the determined threshold value may be stored together with a weight of the deep learning model.

According to another exemplary embodiment of the present disclosure, there may be disclosed a server that performs the method of generating a model for predicting heart disease. The server may include a memory that stores one or more instructions and a processor that executes one or more instructions stored in the memory, wherein the processor may perform the described method of generating the model for predicting heart disease by executing the one or more instructions.

According to another exemplary embodiment of the present disclosure, there may be disclosed a computer-readable recording medium. The computer-readable recording medium may perform the method of generating the model for predicting heart disease while being combined with a computer, which is hardware.

Other specific details of the present disclosure are included in the detailed description and drawings.

According to various exemplary embodiments of the present disclosure, an accuracy of predicting a patient's heart disease can be improved by extracting ROI electrocardiogram data from a patient's electrocardiogram data and then applying the extracted ROI electrocardiogram data to a deep learning model.

In addition, the present disclosure can provide an effect of allowing a patient to identify his or her health condition and to prevent a heart disease in advance by predicting the risk of future heart disease by utilizing a plurality of machine learning models. Through this, an opportunity to protect patients' lives and improve treatment outcomes can be provided by enabling early detection and rapid response to heart disease.

In addition, the present disclosure can provide an effect of increasing an efficiency of screening and reducing opportunity costs in the medical field by drastically reducing the physical time of medical staff who have to analyze electrocardiogram data one by one through an automated AI-based process.

Additionally, the present disclosure can further improve an accuracy of predicting the risk of heart disease by performing ensembles by using a plurality of tree-based machine learning models in order to ensemble HRV feature information, latent vectors extracted from embedding models, and metadata such as a patient's age.

The effects of the present disclosure are not limited to the effects mentioned above, and other effects not mentioned will be clearly understood by those skilled in the art from the following description.

BRIEF DESCRIPTION OF THE DRAWINGS

FIG. 1 is a view showing a configuration of a computing device which performs a method of generating a model for predicting heart disease according to an embodiment exemplary of the present disclosure.

FIG. 2 is a view showing a flowchart of a method of training a deep learning model for predicting heart disease according to an exemplary embodiment of the present disclosure.

FIG. 3 is a view showing a method of classifying electrocardiogram data according to an exemplary embodiment of the present disclosure.

FIG. 4 is a view showing a method for segmenting electrocardiogram data into discrete heartbeats according to an exemplary embodiment of the present disclosure.

FIGS. 5A to 5C are views showing a type of a deep learning model according to an exemplary embodiment of the present disclosure.

FIG. 6 is a view showing steps of training a deep learning model according to an exemplary embodiment of the present disclosure.

FIG. 7 is a view showing a flowchart of a method for predicting heart disease by using a trained deep learning model according to an exemplary embodiment of the present disclosure.

FIG. 8 is a view showing steps of predicting heart disease by using a deep learning model according to an exemplary embodiment of the present disclosure.

FIG. 9 is a view showing an exemplary flowchart related to a method of generating a model for predicting heart disease according to an exemplary embodiment of the present disclosure.

FIG. 10 is a view showing an exemplary flowchart of a preprocessing process on a plurality of electrocardiogram data according to an exemplary embodiment of the present disclosure.

FIG. 11 is a view showing an exemplary flowchart of a process of performing embedding with respect to latent vectors according to an exemplary embodiment of the present disclosure and constructing a vector database according to the performance result.

FIG. 12 is a view showing an exemplary flowchart of a process of generating an embedding model according to an exemplary embodiment of the present disclosure.

FIG. 13 is an exemplary view for explaining a process of generating an embedding model from a self-reconstruction model and a trained self-reconstruction model according to an exemplary embodiment of the present disclosure.

FIG. 14 is a view showing an exemplary flowchart for explaining a process of generating a model for predicting the risk of heart disease through ensemble learning of a plurality of tree-based models according to an exemplary embodiment of the present disclosure.

DETAILED DESCRIPTION OF THE DISCLOSURE

Specific structural or functional descriptions of exemplary embodiments are disclosed for illustrative purposes only and may be modified and implemented in various forms. Accordingly, the actual implemented form is not limited to the specific exemplary embodiments disclosed, and the scope of the present specification includes modifications, equivalents, or alternatives included in the technical idea described in the exemplary embodiments.

Terms such as a first or a second may be used to describe various components, but these terms should be interpreted only to distinguish one component from another. For example, a first component may be referred to as a second component, and similarly, a second component may also be referred to as a first component.

When it is mentioned that a component is "connected" to another component, it should be understood that the component may be directly connected to or linked to that other component but other components may exist in the middle.

Singular expressions include plural expressions unless the context clearly indicates otherwise. In this document, each of the phrases such as "A or B", "at least one of A and B", "at least one of A or B", "A, B or C", "at least one of A, B and C", and "at least one of A, B, or C" may include any of the items listed together in the corresponding phrase, or any possible combination thereof. In this specification, terms such as "include" or "have" are intended to designate that the described features, numbers, steps, actions, components, parts or combinations thereof exist, and should be understood not to preclude the existence or possibility of addition of one or more other features or numbers, steps, actions, components, parts or combinations thereof.

Unless otherwise defined, all terms used herein, including technical or scientific terms, have the same meaning as commonly understood by those skilled in the art. Terms such as those defined in a generally used dictionary should be interpreted as having a meaning consistent with the meaning in the context of the relevant technology and are not interpreted in an ideal or overly formal sense unless explicitly defined herein.

Hereinafter, exemplary embodiments will be described in detail with reference to the accompanying drawings. In the description with reference to the accompanying drawings, the same elements regardless of the reference numerals are assigned with the same reference numerals, and redundant descriptions thereof will be omitted.

The present disclosure may obtain a plurality of electrocardiogram data corresponding to a plurality of patients, and generate a model for predicting heart disease on the basis of the plurality of obtained electrocardiogram data. In an exemplary embodiment, the model for predicting heart disease may be a neural network model that extracts important patterns and features from electrocardiogram data, on the basis of which predicts a possibility of heart disease. The computing device of the present disclosure may process the plurality of electrocardiogram data into a form suitable for training, and may train the model for predicting heart disease using the processed data.

According to an exemplary embodiment, the present disclosure may generate and provide a deep learning model and a model for predicting the risk of heart disease as a model for predicting heart disease. In an exemplary embodiment, the present disclosure may generate and provide various prediction models by processing the plurality of electrocardiogram data into various forms and training the neural network in different ways. Hereinafter, the method of generating models for predicting heart disease will be described in detail with reference to various drawings.

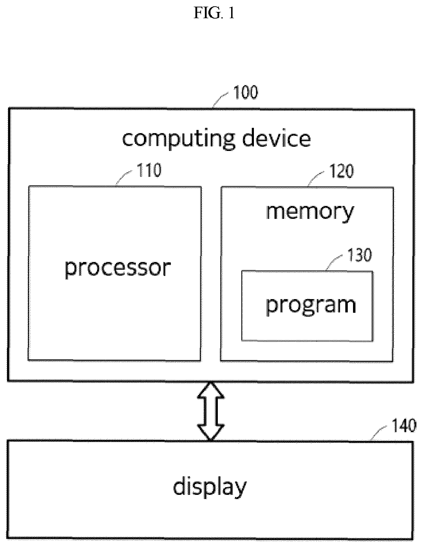

FIG. 1 is a view showing a configuration of a computing device which performs a method of generating a model for predicting heart disease according to an embodiment exemplary of the present disclosure.

As shown in FIG. 1, the computing device 100 may include one or more processors 110 and a memory 120 for loading or storing a program 130 executed by the processor 110. The components included in the computing device 100 of FIG. 1 may be merely examples, and a person skilled in the art to which the present disclosure pertains will recognize that other general components may be further included in addition to the components shown in FIG. 1.

The processor 110 may control the overall operation of each component of the computing device 100. The processor 110 may be configured to include at least one of a central processing unit (CPU), a microprocessor unit (MPU), a microcontroller unit (MCU), a graphics processing unit (GPU), a natural processing unit (NPU), a digital signal processor (DSP), or any type of processors well known in the art of the present disclosure. In addition, the processor 110 may perform operations for at least one application or program for executing a method/operation according to various exemplary embodiments of the present disclosure. The computing device 100 may include one or more processors.

The memory 120 may store one or a combination of two or more of various data, instructions, and information used by components (e.g., a processor 110) included in the computing device 100. The memory 120 may include a volatile memory and/or a nonvolatile memory.

The program 130 may include one or more actions in which methods/operations according to various exemplary embodiments of the present disclosure are implemented, and may be stored in the memory 120 as a software form. Herein, the actions may correspond to commands implemented in the program 130. For example, the program 130 may include instructions to perform an action of classifying electrocardiogram data obtained from a plurality of patients into a training data set, a validation data set, and a test data set, an action of obtaining ROI electrocardiogram data segmented into a predefined window size from each of electrocardiogram data classified into the training data set, the validation data set, and the test data set, an action of training the deep learning model to predict a heart disease class of each first ROI electrocardiogram data in response to inputting the first ROI electrocardiogram data obtained from electrocardiogram data included in the training data set into the deep learning model for predicting a patient's heart disease, and an action of determining a threshold value for distinguishing the heart disease class by applying second ROI electrocardiogram data obtained from electrocardiogram data included in the validation data set to the trained deep learning model.

According to an exemplary embodiment, the ROI electrocardiogram data segmented into the predefined window sizes may mean a discrete heartbeat, but is not limited thereto. Hereinafter, for convenience of explanation according to an exemplary embodiment, discrete heartbeats may be described as an example for the ROI electrocardiogram data segmented into the predefined window size, but the type of ROI electrocardiogram data applied to each exemplary embodiment is not limited thereto.

When the program 130 is loaded into the memory 120, the processor 110 may perform methods/operations according to various exemplary embodiments of the present disclosure by executing a plurality of actions for implementing the program 130.

An execution screen of the program 130 may be displayed through a display 140. In the case of FIG. 1, the display 140 may be expressed as a separate device connected to the computing device 100, but in the case of the computing device 100 such as a user-portable terminal such as a smartphone, a tablet, or the like, the display 140 may be a component of the computing device 100. The screen expressed on the display 140 may be before inputting information into the program or a result of executing the program.

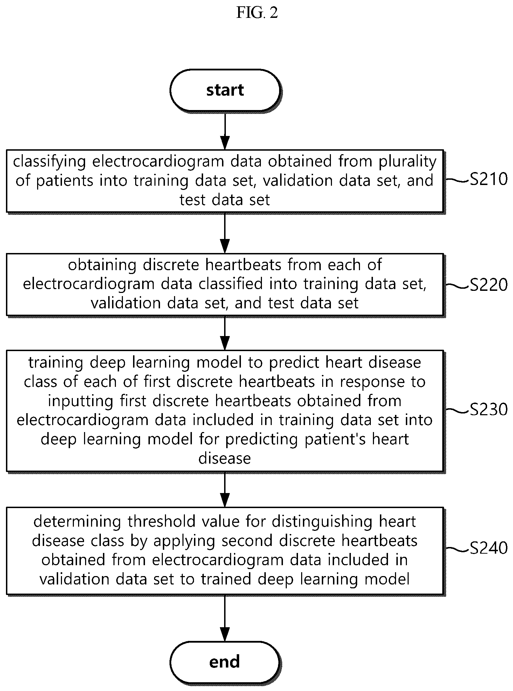

FIG. 2 is a view showing a flowchart of a training method of a deep learning model for predicting heart disease according to an exemplary embodiment of the present disclosure.

The training method of the deep learning model shown in FIG. 2 may be performed by the processor of the computing device shown FIG. 1. In the step 210, the processor may classify electrocardiogram data obtained from the plurality of patients into the training data set, the validation data set, and the test data set. First, electrocardiogram data obtained from the plurality of patients may be classified into groups of a true-normal sinus rhythm (T-NSR), an atrial fibrillationnormal sinus rhythm (AF-NSR), and a clinically important arrhythmia-normal sinus rhythm (CIA-NSR). In this case, electrocardiogram data obtained from the plurality of patients may be 10-second 12-lead electrocardiogram data, but the type of such electrocardiogram data is only one example and is not limited to the above example.

Meanwhile, the clinically important arrhythmia (CIA) may include atrial arrhythmia, ventricular arrhythmia, atrial fibrillation, and bundle branch block (BBB). Atrial arrhythmia may be an arrhythmia originating in the atrium, may refer to arrhythmia sustained for more than 30 seconds or non-sustained within 30 seconds, and may include atrial premature complexes, or sustained/non-sustained atrial rhythm. Ventricular arrhythmia may be an arrhythmia originating in the ventricle, may refer to the arrhythmia sustained for more than 30 seconds or non-sustained within 30 seconds, and may include ventricular premature complexes, or sustained/non-sustained ventricular arrhythmia. Atrial fibrillation may refer to an arrhythmia in which there is no regular electrical signal and contraction of the atrium, resulting in irregular ventricular contractions. Finally, BBB may refer to an arrhythmia that shows a characteristic electrocardiogram pattern since blocking the signal transmission of the right bundle or left bundle that transmits heart signals through the ventricles.

The T-NSR group may consist of electrocardiograms from patients who have no history of atrial fibrillation or arrhythmia and have three or more normal sinus rhythm electrocardiograms per year. The AF-NSR group may consist of electrocardiograms from patients with a normal sinus rhythm electrocardiogram paired with an atrial fibrillation or atrial flutter electrocardiogram that occurs from the corresponding normal sinus rhythm electrocardiogram within 14 days. Likewise, the CIA-NSR group may consist of electrocardiograms from patients with a normal sinus rhythm electrocardiogram paired with an arrhythmia electrocardiogram that occurs from the corresponding normal sinus rhythm electrocardiogram within 14 days.

The processor may classify electrocardiogram data belonging to these distinct T-NSR group, AF-NSR group, and CIA-NSR group into the training data set, the validation data set, and the test data set applicable to the deep learning model for predicting heart disease.

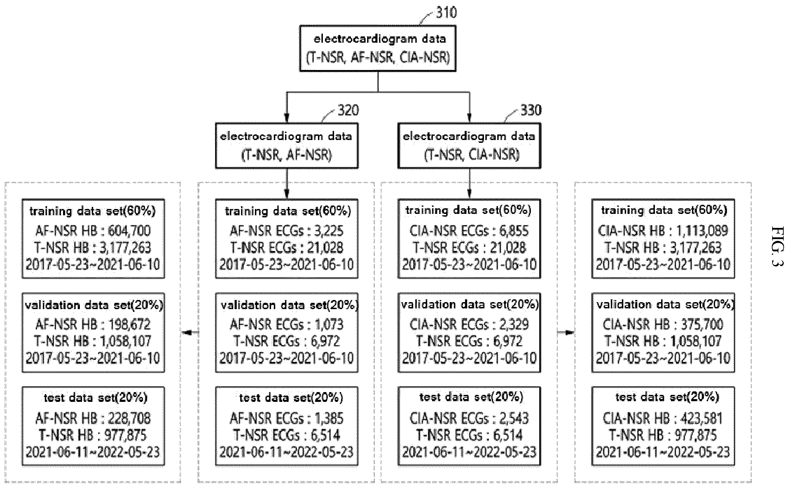

For example, FIG. 3 is a view showing a method of classifying electrocardiogram data according to an exemplary embodiment of the present disclosure. The processor may classify electrocardiogram data 310 belonging to the T-NSR group, the AF-NSR group, and the CIA-NSR group according to heart disease to be predicted. For example, the processor may classify the electrocardiogram data 310 into electrocardiogram data 320 belonging to the T-NSR group and the AF-NSR group in order to predict atrial fibrillation, and classify the same into electrocardiogram data 330 belonging to the T-NSR group and the CIA-NSR group in order to predict arrhythmia.

Thereafter, the processor may classify the electrocardiogram data 320, 330 classified by each heart disease into the training data set, the validation data set, and the test data set on the basis of an arbitrary date on which the electrocardiogram data 320, 330 are generated.

For example, as shown in FIG. 3, when electrocardiogram data 320, 330 are obtained from 2017-05-23 to 2022-05-23, the processor may classify on the basis of an arbitrary date (e.g., 2021-06-11) the electrocardiogram data before the corresponding date among the electrocardiogram data 320, 330 into the training data set and the validation data set at a certain ratio, and classify the electrocardiogram data after the corresponding date (including that date) into the test data set. In the example of FIG. 3, the electrocardiogram data 320, 330 may be classified at a ratio of the training data set (60%), the validation data set (20%), and the test data set (20%), but such a classification ratio is only one example and is not limited to the above example.

In the step 220, the processor may obtain discrete heartbeats from each of the electrocardiogram data classified into the training data set, the validation data set, and the test data set. The processor may perform preprocessing on the 10-second 12-lead electrocardiogram data classified into the training data set, the validation data set, and the test data set in order to obtain accurate and reliable data.

More specifically, the processor may receive the electrocardiogram data classified into the training data set, the validation data set, and the test data set in the form of an eXtensible markup language (XML) file as an input. The processor may parse a structured data part, such as a patient's name, age, and gender, and an unstructured data part consisting of continuous signals from the input data. Thereafter, the processor may perform denoising preprocessing on the unstructured data part consisting of continuous signals, and may distinguish a key marker of the heartbeat. The processor may obtain discontinuous discrete heartbeats from the unstructured data consisting of continuous signals through the key marker of the heartbeat distinguished in this way.

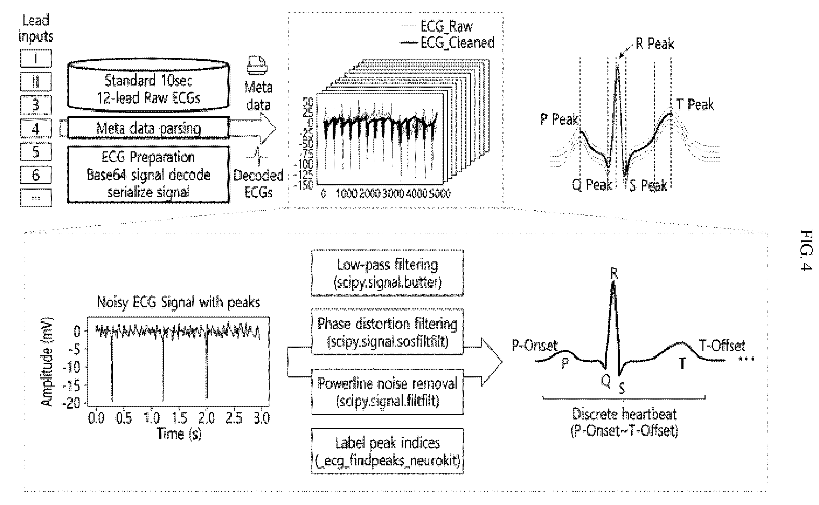

For example, referring to FIG. 4, the processor may decode the 10-second 12-lead electrocardiogram data using Base64 encryption and then pass the same through the IIR Butterworth SOS filter and the powerline noise filter with a moving average kernel for denoising and cleansing. Then the processor may fractionize the denoised 10-second 12-lead electrocardiogram data into discrete heartbeats using the QRS peak detection algorithm.

As a result, as shown in FIG. 3, the processor may obtain a plurality of discontinuous discrete heartbeats from a single 10-second 12-lead electrocardiogram data, and the discrete heartbeats obtained in this way may be used to train the deep learning model for predicting heart disease more accurately.

In the step 230, the processor may train the deep learning model to predict the heart disease class of each of the first discrete heartbeats in response to inputting the first discrete heartbeats obtained from the electrocardiogram data included in the training data set into the deep learning model for predicting a patient's heart disease.

In this case, the processor may train any one of the deep learning models of ResNet-18, Conv1D including LSTM (Long Short-Term Memory), and Conv1D including a transformer to predict the heart disease class of each of the first discrete heartbeats.

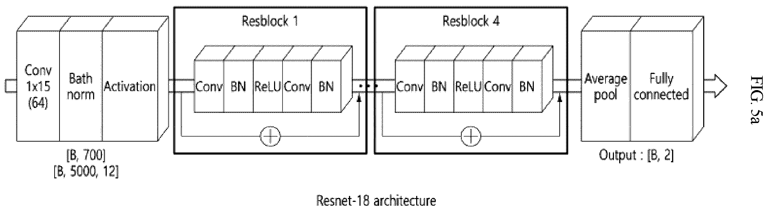

For example, ResNet-18 as shown in FIG. 5A may be a deep learning model capable of extracting essential features of an input by using a convolution operation such as various convolutional neural networks (CNN). In order to solve the vanishing gradient problem of the CNN architecture, ResNet-18 may perform residual learning through a skip connection, a method in which input data is directly connected to the output layer by skipping multiple layers of the network as shown in FIG. 5A.

Since the deep learning model of ResNet-18 requires a fixed-length input, the processor may fix the length of discrete heartbeats to the average length of all the discrete heartbeats. For example, when the average length of all the discrete heartbeats is 700, the processor may perform slicing for the discrete heartbeats having a heartbeat length longer than 700 and perform zero-padding for the discrete heartbeats having a heartbeat length shorter than 700 such that the length is fixed to 700.

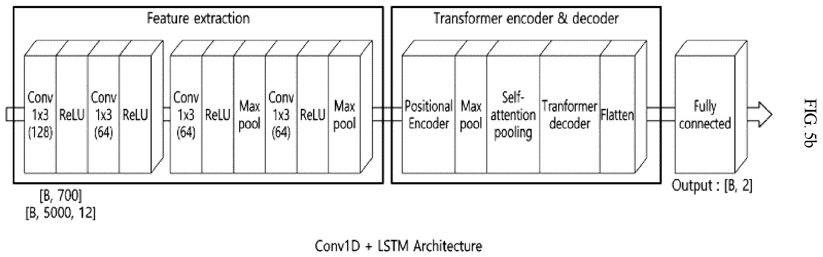

As another example, Conv1D including the LSTM as shown in FIG. 5B may capture all local temporal patterns and long-range temporal patterns in sequential data. In this case, the Conv1D layer may be good at detecting the local temporal pattern, and the LSTM layer may be excellent at modeling long-term dependencies.

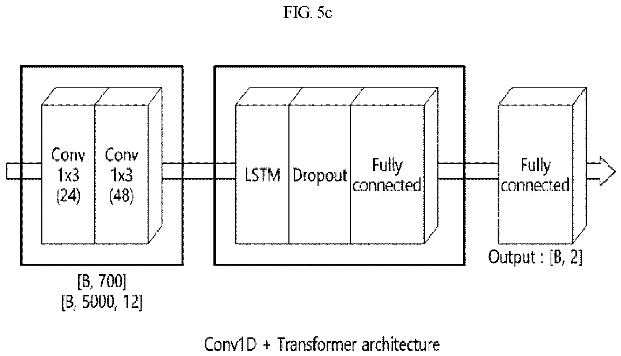

As another example, Conv1D including the transformer as shown in FIG. 5C may capture all the local patterns and the global dependency of the input data. In this case, the transformer layer may be suitable for modeling the global dependency, and the Conv1D layer may be effective for detecting the local patterns. Unlike ResNet-18 with the input length fixed, Conv1D including the transformer may have an advantage of accommodating various input sizes.

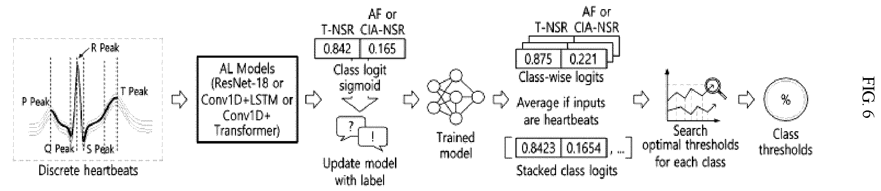

Meanwhile, FIG. 6 is a view showing steps of training a deep learning model according to an exemplary embodiment of the present disclosure. Referring to FIG. 6, the processor may optimize the parameters of the deep learning model by using the binary cross entropy having a log loss and the AdamW optimizer having an initial learning rate of 0.0001. In this case, the binary cross entropy may be a loss function that reduces the difference between a predicted result and an actual correct answer in training the deep learning model, and the AdamW optimizer may be an algorithm involved in actually updating the deep learning model on the basis of such a loss function. The processor may obtain a probability value in the range of 0 to 1 for the heart disease class of each of the first discrete heartbeats by applying a sigmoid function to the output of the deep learning model optimized by this binary cross-entropy and AdamW optimizer.

In the step 240, the processor may determine a threshold value for distinguishing the heart disease class by applying the second discrete heartbeats obtained from the electrocardiogram data included in the validation data set to the trained deep learning model.

More specifically, as shown in FIG. 6, the processor may collect a probability value for each of the discrete heartbeats separated from the same electrocardiogram data for each of the second discrete heartbeats, and derive a final probability score by averaging the probability values for all the collected second discrete heartbeats, thereby fine-tuning an optimal threshold value for distinguishing the heart disease class on the basis of the derived final probability score.

In this case, the optimal threshold value may be obtained by achieving the best F1 score in the validation data set for the heart disease class, i.e., the T-NSR and AF-NSR class or the T-NSR and CIA-NSR class by applying a threshold value between 0 and 1 in units of 0.01.

In this case, the F1 score may be defined as shown in Equation 1 below.

[Equation 1]

$$F1\ score = \frac{2}{\left(\frac{1}{Precision} + \frac{1}{Recall}\right)}$$

Herein, *Precision* is $\frac{True\ Positive}{True\ Positive + False\ Positive}$, and *Recall* is $\frac{True\ Positive}{True\ Positive + False\ Negaive}$.

Herein, *Precision* may represent the proportion of predicted positives that are correctly classified, and may indicate how accurate the detection results are, that is, how much of the detection results contain real objects. *Recall* may represent the proportion of true positives that are correctly classified, and may be an indicator of representing how well real target objects are captured without missing any. In addition, True *Positive* and *True Negative* may refer to samples that are classified as true positive and true negative, respectively, and *False Positive* and *False Negaive* may refer to samples that are classified as false positive and false negative, respectively.

The processor may store the optimal threshold values for such heart disease class along with the weights of the deep learning model.

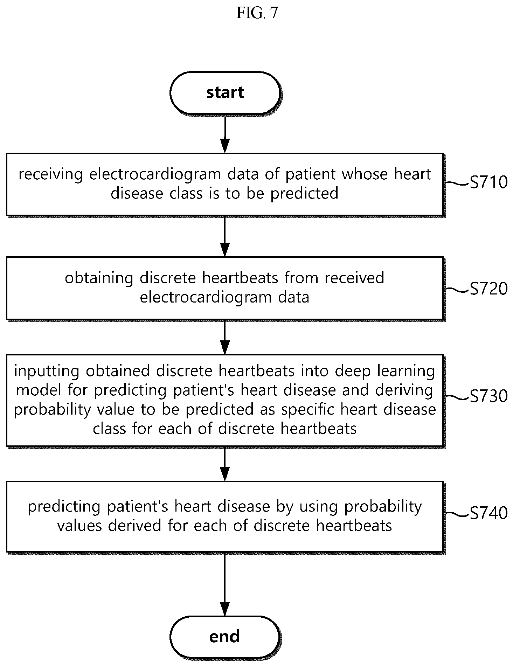

FIG. 7 is a view showing a flowchart of a method for predicting heart disease by using a trained deep learning model according to an exemplary embodiment of the present disclosure.

The method for predicting heart disease by using the deep learning model shown in FIG. 7 may be performed by the processor of the computing device shown in FIG. 1. The processor may receive electrocardiogram data of a patient whose heart disease class is to be predicted as in the step 710 and may obtain discrete heartbeats from the received electrocardiogram data as in the step 720. In this case, the received electrocardiogram data and the obtained discrete heartbeats may correspond to the test data set shown in FIG. 3.

In the step 730, the processor may input the obtained discrete heartbeats into the deep learning model for predicting a patient's heart disease and then derive a probability value to be predicted as a specific heart disease class for each of the discrete heartbeats.

In the step 740, the processor may predict a patient's heart disease by using the probability values derived for each of the discrete heartbeats. More specifically, the processor may distinguish the heart disease class of the patient by comparing the average of the probability values derived for each of all the discrete heartbeats with a threshold value.

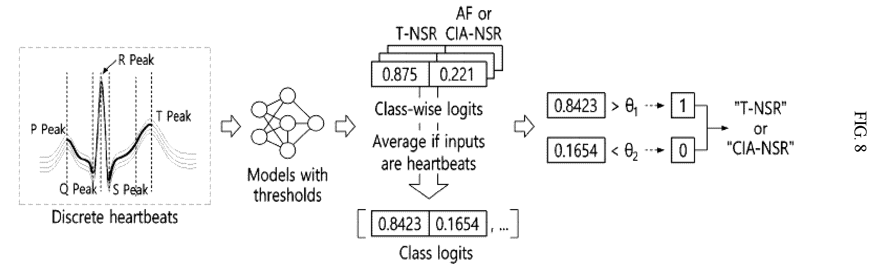

For example, FIG. 8 is a view showing steps of predicting heart disease by using a deep learning model according to an exemplary embodiment of the present disclosure. Referring to FIG. 8, the processor may derive the probability value for each of the discrete heartbeats after loading the weights of the trained deep learning model and the threshold value. The processor may obtain an average value of the probability values of all the derived discrete heartbeats in this way.

For example, the processor may determine the corresponding patient's heart disease class as a normal state, that is, T-NSR when the T-NSR Logit value, which is the average of probability values to be determined as T-NSR, is greater than a first threshold ($\theta_1$) and the CIA-NSR Logit value, which is the average of probability **values to** be determined as CIA-NSR, is less than the second threshold ($\theta_2$).

Alternatively, the processor may compare the T-NSR Logit value with the CIA-NSR Logit value and select the larger value as the final prediction when the T-NSR Logit value is greater than the first threshold ($\theta_1$) and the CIA-NSR Logit value is greater than the second threshold ($\theta_2$). For example, when the CIA-NSR Logit value is greater than the T-NSR Logit value, the corresponding patient's heart disease class may be determined as CIA-NSR where arrhythmia is likely to occur.

In addition, the processor may compare the T-NSR Logit value and the CIA-NSR Logit value and select the larger value as the final prediction when the T-NSR Logit value is less than the first threshold ($\theta_1$) and the CIA-NSR Logit value is less than the second threshold ($\theta_2$).

Finally, when the T-NSR Logit value is less than the first threshold ($\theta_1$) and the CIA-NSR Logit value is greater than the second threshold ($\theta_2$), the processor may determine the corresponding patient's heart disease class as a CIA-NSR where the arrhythmia is likely to occur.

The four methods of distinguishing such a heart disease class may be applied equally to distinguishing T-NSR and AF-NSR.

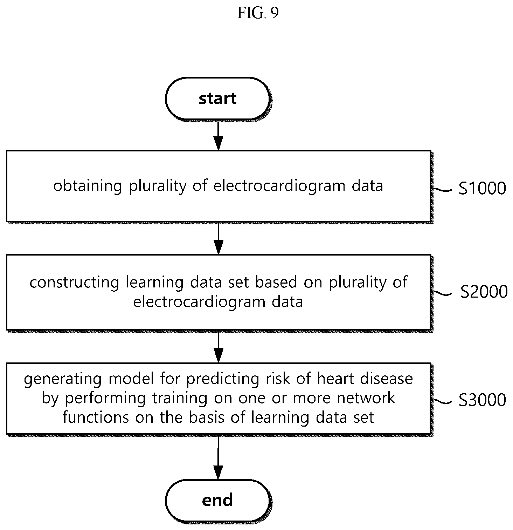

Hereinafter, a method of utilizing the HRV feature information, latent vectors, cluster information corresponding to latent vectors, and metadata related to an exemplary embodiment of the present disclosure and a method of predicting heart disease by ensembling a plurality of tree model outputs will be described in detail with reference to FIGS. 9 to 14.

FIG. 9 is a view showing an exemplary flowchart related to a method of generating a model for predicting heart disease according to an exemplary embodiment of the present disclosure.

According to an exemplary embodiment of the present disclosure, the method of generating the model for predicting heart disease may include obtaining a plurality of electrocardiogram data S1000.

According to an exemplary embodiment, the plurality of electrocardiogram data may be obtained in response to a plurality of patients and may be collected in various ways. In an exemplary embodiment, the electrocardiogram data in the present disclosure may mean multi-lead electrocardiogram (ECG) data related to evaluating a heart condition by recording the electrical activity of the heart. The multi-lead electrocardiogram data may include cardiac electrical signals obtained through various inductions (i.e., leads), and through this may provide comprehensive information about the heart's rhythm, electrical conduction, and other cardiac functions.

In an exemplary embodiment, the plurality of electrocardiogram data obtained from the plurality of patients may include normal electrocardiogram data, electrocardiogram data related to arrhythmia, and electrocardiogram data for other heart conditions. In a specific exemplary embodiment, the electrocardiogram data obtained from the plurality of patients may be classified into groups of true-normal sinus rhythm (T-NSR), Afib-normal sinus rhythm (AF-NSR), and clinically important arrhythmia-normal sinus rhythm (CIA-NSR). In this case, the electrocardiogram data obtained from the plurality of patients may be a 10-second 12-lead electrocardiogram data, but the type of such electrocardiogram data is only one example and is not limited thereto.

In an exemplary embodiment, obtaining the plurality of electrocardiogram data may be receiving or loading data stored in the memory 120. Obtaining the plurality of electrocardiogram data may be receiving or loading the plurality of electrocardiogram data from another storage medium, another computing device, or a separate processing module within the same computing device on the basis of a wired/wireless communication means. For example, a user (e.g., a patient, or a medical provider) may access the computing device 100 through a user terminal to be provided with a user interface for predicting heart disease from the computing device 100 and may transmit the electrocardiogram data to the computing device 100 in a manner of dragging and dropping the electrocardiogram data on the provided user interface.

According to various exemplary embodiments, the computing device 100 of the present disclosure may perform data augmentation for the plurality of obtained electrocardiogram data. Specifically, the computing device 100 may augment the learning data for training the neural network by performing data augmentation by utilizing the lead pairs.

In detail, each of the electrocardiogram data may be obtained for each lead. For example, the electrocardiogram data may be collected in various lead configurations such as 1-lead, 3-lead, and 12-lead, which is used to evaluate a more comprehensive heart condition by recording electrical activity in various parts of the heart.

For example, in the case of the 12-lead signal, many signals may be obtained in a relatively short period of time, but the total amount of data may be limited. On the other hand, the 1-lead signal may be recorded for a longer period of time, but the variety of data may be limited. When these two types of data are used together, more accurate heart condition evaluation may be possible, but the number of such data pairs may not be sufficient in practice.

To solve this problem, the computing device 100 of the present disclosure may generate a reconstruction model that generates other lead data on the basis of one lead data. The reconstruction model may play a role in generating multiple lead data on the basis of receiving one lead data as input. This process may have an effect of expanding one lead data to 3 leads or more leads (12 leads) and augmenting the learning data as if there is more lead data.

Training of the reconstruction model may include a process of training the correlation between one lead and multiple lead data. To this end, the reconstruction model may be trained by using pair data in which one lead and multiple lead data all exist. In the training process, the model may learn how to reproduce multiple lead data from one lead data, and may learn the features of one lead data and the pattern of multiple lead signals inferred therefrom.

After the model training is completed, the reconstruction model may generate multiple lead signals with only one lead data, which can actually compensate for the lack of multiple lead data. This may increase the diversity of data and improve the generalization performance of the model. The multiple lead data (i.e., reinforced multi-lead data) generated in this way may be used to train a neural network along with existing multiple lead data, so the model may learn more diverse lead configurations and improve the prediction performance for various heart conditions.

As a result, data augmentation through reconstruction models may substantially increase the amount of learning data, thereby improving the prediction accuracy of the model and enhancing the reliability of the model for predicting heart disease. This may help medical professionals more accurately diagnose patients' health conditions and establish more effective treatment and prevention plans.

According to an exemplary embodiment of the present disclosure, the method of generating the model for predicting heart disease may include constructing the learning data set based on the plurality of electrocardiogram data S2000.

In an exemplary embodiment, constructing the learning data set through the plurality of electrocardiogram data may be a process of collecting and configuring various data in order to learn important features and patterns of each electrocardiogram data. Through this, a data set including various cases and patterns necessary for predicting heart disease may be generated.

According to an exemplary embodiment, the learning data set may include various data related to the prediction of heart disease, and may include the training data set for training a neural network model, the validation data set for evaluating and optimizing the performance of the model, and the test data for evaluating the generalization performance of the model.

In an exemplary embodiment, the learning data set may include a first learning data set and a second learning data set classified for different training purposes. The first learning data set and the second learning data set may be data classified for training neural network models different from each other. The first learning data set and the second learning data set may be classified by the computing device 100.

For example, the first learning data set may be utilized in the training process of the embedding model, and the corresponding data set may focus on extracting and representing key features of electrocardiogram data. The second learning dataset may be used to train the model for predicting the risk of heart disease, and the corresponding data set may be used for calibration and performance evaluation for improving the accuracy of the prediction model.

Specifically, the first learning data set may include data for training corresponding to the process of transforming features of electrocardiogram data into a feature space. The first learning data set may be data for the electrocardiogram representation learning, and may be utilized to extract and embed various features and patterns of electrocardiogram signals. In addition, the second learning data set may include data for calibration related to predicting cardiac risk. That is, the second learning data set may be data for calibrating cardiac risk, and may be utilized to more accurately match the risk predicted by the model.

According to an exemplary embodiment, the constructing of the learning data set may include performing preprocessing on the plurality of electrocardiogram data.

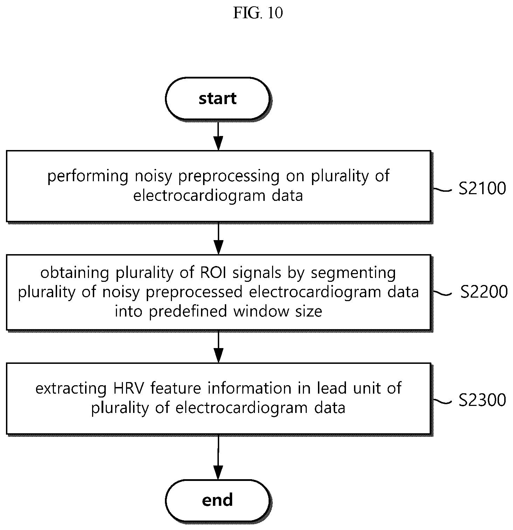

FIG. 10 is a view showing an exemplary flowchart of a preprocessing process on a plurality of electrocardiogram data according to an exemplary embodiment of the present disclosure.

Referring to FIG. 10, the preprocessing method on the plurality of electrocardiogram data may include performing noisy preprocessing on the plurality of electrocardiogram data S2100, obtaining a plurality of ROI signals by segmenting a plurality of noisy preprocessed electrocardiogram data into a predefined window size S2200, and extracting heart rate variability (HRV) feature information in a lead unit of the plurality of electrocardiogram data S2300. In an exemplary embodiment, the HRV feature information may include indicative information on variability between heart rate intervals.

To explain in more detail, first, preprocessing for removing noise from the plurality of obtained electrocardiogram data may be performed in the step S2100. Herein, the preprocessing for removing noise may include a low-pass filtering and a noise removal, which is for removing baseline noise of the electrocardiogram signal. More specifically, the low-pass filtering may enhance the purity of the signal by removing high-frequency components from the electrocardiogram data through a frequency band-limited method, and the noise removal may further clarify important signal components by reducing unnecessary noise in the electrocardiogram data by utilizing a time series analysis technique. This preprocessing process may minimize distortion of the electrocardiogram signal and contribute to improving signal quality for more accurately evaluating heart activity.

In addition, according to an exemplary embodiment, the computing device 100 may perform data augmentation to increase the diversity and robustness of model training. The computing device 100 may perform data augmentation in a way of simulating various noise patterns, thereby allowing the model to learn and adapt to various noise situations that may occur in a real environment.

In a specific exemplary embodiment, in the process of preprocessing on electrocardiogram data, various noises such as Gaussian noise, jitter noise, random time warping, scaling, amplitude modulation, and phase shift that may occur in real-world electrocardiogram measurement environments may be artificially added to augment the dataset, thereby enabling the model to learn and adapt to noises that may occur in various environments. This noise addition and removal process may have an advantage of improving the quality of data and enhancing the generalization performance of the model.

Thereafter, in the step S2200, the electrocardiogram data from which noise is removed may be segmented into a predefined window size and organized into a plurality of ROI signals. According to an exemplary embodiment, the ROI signal may be defined as a range including at least 3 to 5 QRS Complexes (heartbeats) in a transformable window size unit including meaningful heartbeats from each of the electrocardiogram data. In an exemplary embodiment, the QRS Complex may represent depolarization of the ventricle during electrical activity of the heart, and may contribute to identifying the start and end of the heartbeat as a key feature in the electrocardiogram. For example, each ROI signal may be segmented into a unit of 30 seconds to 1 minute, which is useful for analyzing an important pattern in the electrocardiogram signal and for identifying the regularity and abnormality of heartbeats.

The computing device 100 may extract the ROI signal and organize the same into data that allows to more accurately analyze key features of the heartbeats. This may play an important role in identifying specific patterns and abnormal signs of the electrocardiogram signal and evaluating the risk of heart disease.

In addition, a process of extracting HRV feature information may be performed in the step S2300. The HRV feature information may be an indicator that measures periodic changes in heartbeat over time, and may be an important signal feature that reflects the activity of the heart. For example, the computing device 100 may obtain the plurality of ROI signals by segmenting electrocardiogram data into units of 30 seconds to 1 minute and may extract HRV features corresponding to each ROI signal. This may be for more accurately evaluating the overall activity of the heart by analyzing data over a longer time range than the existing beat-by-beat analysis.

The computing device 100 may derive HRV features from each ROI signal through a method such as frequency analysis or time domain analysis. In a specific exemplary embodiment, the computing device 100 may extract HRV feature information corresponding to each ROI signal by applying various analysis techniques. For a specific example, the computing device may extract HRV feature information through the evaluation of high-frequency and low-frequency components through spectral analysis and statistical analysis of NN intervals.

Spectral analysis may evaluate heart rate variability in the frequency domain, so that the high frequency component mainly reflects the activity of the parasympathetic nervous system and the low frequency component represents the mixed activity of the sympathetic and parasympathetic nervous systems. This may be useful for identifying the balance of the autonomic nervous system. Statistical analysis of NN intervals may quantify the activity of the autonomic nervous system of the heart by evaluating the variability of beat-to-beat intervals in the time domain. This can be an important indicator for evaluating irregularities in heart rate, stress responses, and heart health conditions.

In an exemplary embodiment, the HRV feature information extracted in a lead unit may include root mean square of successive differences (RMSSD), standard deviations of NN intervals (SDNN), standard deviation of average NN intervals (SDANN), and the like. Specifically, the RMSSD may refer to the square root of the root mean square of the difference between successive heartbeat intervals and may mainly reflect the influence of the parasympathetic nervous system. SDNN may represent the standard deviation of all normal heartbeat intervals and may represent the overall variability of the autonomic nervous system. SDANN may evaluate long-term heartbeat variability by measuring the standard deviation of the average heartbeat interval over a certain period of time. These indicators may play an important role in evaluating the activity of the autonomic nervous system of the heart and comprehensively analyzing the state of heart health. That is, the HRV feature information may be used to reflect various aspects of heart rate variability and evaluate heart rate irregularity, activity of the autonomic nervous system and more and thus may be a basis for predicting the risk of heart disease.

According to an exemplary embodiment of the present disclosure, the method of generating the model for predicting heart disease may include generating the model for predicting the risk of heart disease by performing training on one or more network functions on the basis of the learning data set S3000.

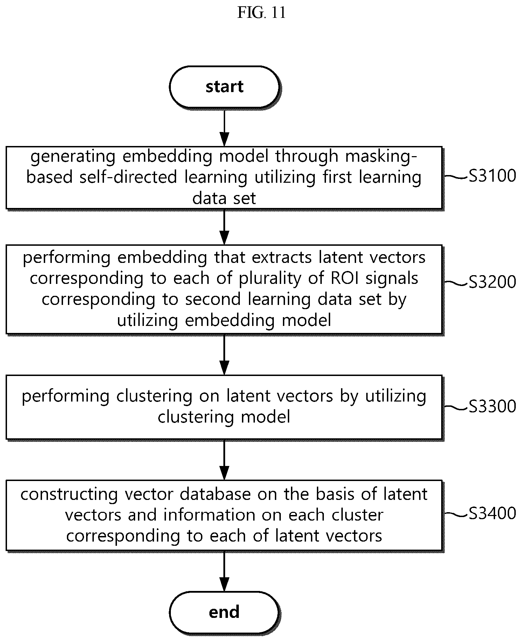

FIG. 11 is a view showing an exemplary flowchart of a process of performing embedding with respect to latent vectors according to an exemplary embodiment of the present disclosure and constructing a vector database according to the performance result.

Referring to FIG. 11, the generating of the model for predicting the risk of heart disease may include generating an embedding model through masking-based self-supervised learning by utilizing the first learning data set S3100, performing the embedding that extracts latent vectors corresponding to each of the plurality of ROI signals corresponding to the second learning data set by utilizing the embedding model S3200, performing clustering on latent vectors by utilizing a clustering model S3300, and constructing a vector database on the basis of latent vectors and information on each cluster corresponding to each of the latent vectors S3400.

To explain in more detail, the computing device 100 may generate an embedding model on the basis of the first learning data set in the step S3100.

The embedding model may be a model trained to represent complex patterns of electrocardiogram data in a low-dimensional space. When the plurality of ROI signals corresponding to electrocardiogram data are inputted, the embedding model may generate a latent vector corresponding to each ROI signal.

Herein, the latent vector may be a low-dimensional vector that compressively represents the important features of the electrocardiogram data. This may be a data representation transformed into a form containing the main information of a signal in order to make it easier to understand and analyze an electrocardiogram signal, which is high-dimensional data. The latent vector may provide useful information for evaluating a heart health condition or detecting signs of disease on the basis of patterns of electrocardiogram data.

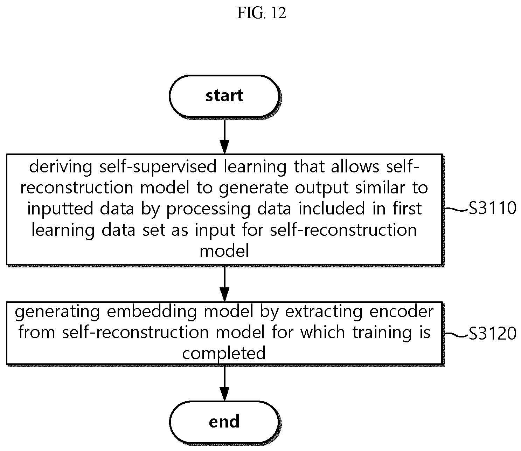

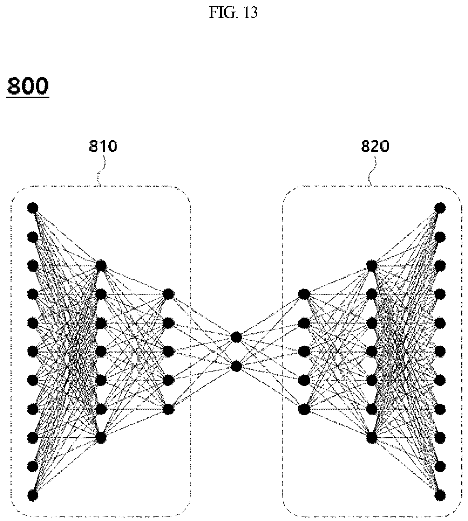

A detailed description of the method of generating the embedding model will be described later with reference to FIGS. 12 and 13.

FIG. 12 is a view showing an exemplary flowchart of a process of generating an embedding model according to an exemplary embodiment of the present disclosure. FIG. 13 is an exemplary view for explaining a process of generating an embedding model from a self-reconstruction model and a trained self-reconstruction model according to an exemplary embodiment of the present disclosure.

Referring to FIG. 12, the generating of the embedding model may include deriving self-supervised learning that allows the self-reconstruction model to generate an output similar to the inputted data by processing the data included in the first learning data set as input for the self-reconstruction model S3110 and generating an embedding model by extracting an encoder from the self-reconstruction model for which training is completed S3120.

The self-reconstruction model may be a model designed to inherently learn important features of data in the process of training through input and output.

In an exemplary embodiment, the self-reconstruction model may be a neural network model that masks a part of inputted data and restores the masked part, and may include an encoder (or a dimension reduction network function) and a decoder (or a dimension recovery network function). In an exemplary embodiment, the self-reconstruction model of the present disclosure may be a Masked AutoEncoder (MAE) that learns important patterns from electrocardiogram data and accurately restores masked signals, but is not limited thereto.

Self-reconstruction models may effectively learn important features of the data through a process of intentionally masking a part of the electrocardiogram signal (specifically, each ROI signal) and reconstructing the entire signal on the basis of the remaining part.

The self-reconstruction model may perform learning in a way that minimizes the restoration error between input and output, and through this, important features of signals may be effectively extracted and represented. Self-reconstruction models may have an advantage in understanding and utilizing the inherent structure of data, especially when data is complex or incomplete.

To explain in more detail, as shown in FIG. 13, the self-reconstruction model 800 may learn important features through a process of dimensionally reducing the input data and then restoring it. The self-reconstruction model may include an encoder (or a dimension reduction network function) 810 and a decoder (or a dimension recovery network function) 820. The self-reconstruction model 800 may transform a high-dimensional data into a low-dimensional latent space through the encoder 810, and in this process, may compress key information of the data and reduce noise. Then, the self-reconstruction model may attempt to restore the compressed low-dimensional representation to an original high-dimensional space by using the decoder 820, thereby being capable of learning and reproducing important patterns of data.

According to an exemplary embodiment, the computing device 100 may induce the self-reconstruction model 800 to train through a masking learning method. In this method, a part of the input data may be intentionally masked, and the model may be trained to restore the masked part. For example, since being trained to be biased toward noise rather than QRS complexes without masking, the self-reconstruction model may be trained to focus on important features through masking. Through this, the model may develop the ability to accurately restore masked information by extracting important patterns and features from the remaining part of the input data. This may have an advantage of operating robustly to noise or incomplete parts of the data.

In addition, it may be possible to understand the context of the data and learn important features in the process of restoring the masked part, which is very useful for analyzing and interpreting complex medical data such as electrocardiogram data. By applying the mask, the model may focus on key signals such as QRS Complex, thereby enabling reliable data analysis for accurate diagnosis and evaluation of heart disease.

That is, the present disclosure may train the self-reconstruction model in a masking learning fashion to focus on important heart signals in order to increase reliability in analyzing and interpreting electrocardiogram data. This may enable the model to understand the context of the data and learn key features, thereby accurately identifying and restoring key signals such as QRS Complex.

The self-reconstruction model may aim to reproduce the entire signals and focus on restoring the masked part after masking a certain meaningful range of the signals. As a specific example, the meaningful range in the entire range of the electrocardiogram signal may be masked and within this masked area, about three QRS complexes may be included. The self-reconstruction model may learn this area intensively, and the loss function may be calculated only for this area. In this way, the model may learn the features of the data centered on the important signals, thereby more accurately understanding and reproducing the overall context of the data.

As a result, the model may operate robustly even in the presence of noise or incomplete parts that occur in electrocardiogram data, and may enable reliable data analysis necessary for early diagnosis and evaluation of heart disease. This approach may minimize the loss of important information in the analysis of electrocardiogram signals and may significantly improve the interpretation of medical data by supporting accurate evaluation of heart conditions.

According to an exemplary embodiment, the self-reconstruction model 800 may include at least one hidden layer, and an odd number of hidden layers may be disposed between input and output layers. The number of nodes in each layer may be reduced from the number of nodes of the input layer to an intermediate layer called a bottleneck layer (encoding), and then may be expanded symmetrically to the reduction from the bottleneck layer to the output layer (symmetrical to the input layer). In this case, the layer of encoder 810 and the layer of decoder 820 may be shown to be symmetrical in FIG. 13, but the present disclosure is not limited thereto, and the layer of the encoder 810 and the node of the layer of the decoder 820 may or may not be symmetrical. The self-reconstruction model 800 may perform nonlinear dimensionality reduction. The number of input layers and output layers may correspond to the number of items of input data remaining after preprocessing of the input data. The number of nodes of the hidden layer included in the encoder 810 in the structure of the self-reconstruction model 800 may have a configuration of decreasing as getting farther away from the input layer. The number of nodes in the bottleneck layer (the layer with the fewest nodes located between the encoder and decoder) may be maintained above a certain number (e.g., more than half of the input layer, etc.) because too few nodes may not transmit enough information.

In an exemplary embodiment, the computing device 100 may train the self-reconstruction model through the plurality of ROI signals corresponding to the first learning data set, through which the model may learn important features and patterns of electrocardiogram data. The trained self-reconstruction model may be applied to each of the plurality of electrocardiogram data and may extract and analyze key features of the electrocardiogram signal while going through a process of masking and restoring a part of the inputted data.

In summary, the computing device 100 may induce learning by using the masking learning method of the self-reconstruction model to secure high reliability in the analysis and interpretation of electrocardiogram data. In this process, the self-reconstruction model may focus on the main signals such as the QRS Complex and learn the unique distribution of the heartbeat. While reproducing the original signal of the electrocardiogram data, the self-reconstruction model may effectively learn important features that the electrocardiogram data itself has such as the peak of the QRS Complex.

In the subsequent step S3120, the embedding model may be generated by extracting the encoder from the self-reconstruction model in which training is completed. Since the encoder part in the trained self-reconstruction model is trained (i.e., pre-trained through the first learning data set) to compress important features of high-dimensional data and remove noise, a low-dimensional latent vector may be outputted in response to the inputted data (e.g., the plurality of ROI signals included in the second learning data set).

According to an exemplary embodiment, in the case of the generated embedding model extracting and representing the features of the data, the similarity between inputted data may be effectively reflected in the latent space. For example, the embedding model in response to similar input data may output a latent vector on a similar latent space. For example, the second latent vector of the second ROI signal similar to the first ROI signal may be located in a latent space similar to the first latent vector corresponding to the first ROI signal. That is, it may be disposed at a position close to the latent space.

On the contrary, when the input data are different from each other (i.e., when there is a large difference), the embedding model may place the data at a location far from each other in the latent space. Accordingly, similarities and differences between data may be clearly identified on the basis of characteristic patterns in the electrocardiogram data, which may be utilized in the process of diagnosing and evaluating heart disease.

That is, the present disclosure may induce the self-reconstruction model to be self-trained by utilizing the first learning data set, and form an embedding model by extracting an encoder from the self-reconstruction model in which training is completed.

After generating the embedding model by utilizing the first learning data set, the computing device 100 may perform embedding that extracts latent vectors corresponding to each of the plurality of ROI signals corresponding to the second learning data set by utilizing the embedding model.

More specifically, each ROI signal included in the second learning data set may be provided as an input into the embedding model by computing device 100, and the embedding model may output a low-dimensional latent vector by compressing the key features of the corresponding signals. The latent vector may be a concise representation of the complex pattern of ROI signals, and may reflect the similarity and difference between each ROI signal in the latent space. For example, signals with similar patterns among the ROI signals of the electrocardiogram data included in the second learning data set may be outputted as latent vectors at close locations in the latent space.

Thereafter, in the step S3300, the computing device 100 may perform clustering on latent vectors by utilizing the clustering model.

According to an exemplary embodiment, the computing device 100 may perform clustering on the latent vectors by utilizing the clustering model.

For example, the clustering model may be a K-Nearest Neighbors (KNN) model, which is a model trained to perform clustering by utilizing the latent vectors extracted by the embedding model.

In an exemplary embodiment, the clustering model may perform clustering according to a similarity distance between latent vectors corresponding to each of the plurality of ROI signals.

To explain in more detail, the clustering process may calculate the distance or similarity between each latent vector and then determine how close or similar these vectors are. Herein, the distance may be generally calculated by using a measurement method such as Euclidean distance or cosine similarity.

For example, the distance between a latent vector A extracted from a specific ROI signal and a latent vector B extracted from another ROI signal may be calculated to evaluate how similar these two vectors are. When the distance between A and B is close, these two vectors may be considered to represent electrocardiogram signals containing the same or similar patterns. The clustering model may group latent vectors into several clusters on the basis of such a distance information.

In the case of the KNN model, the clustering may be performed in a way of finding the K nearest neighbor vectors of a specific vector and enabling the cluster to which those neighbor vectors belong to include the corresponding vector.

For example, when K is 3, the three nearest neighbor vectors of the latent vector A may be found and A may be assigned to the cluster to which most of those neighbors belong among clusters to which the neighbors belong. This process may be repeated for all latent vectors, and finally, vectors that share similar features may be grouped into the same cluster.

This process may be useful for classifying various patterns of electrocardiogram data and analyzing how each pattern is related to a specific type or risk level of heart disease. For example, when a certain cluster contains a signal related to the irregularity of the heartbeat, the corresponding cluster may be utilized as an indicator of a disease such as arrhythmia.

For a more specific example, when performing clustering for each latent vector by utilizing the clustering model, four clusters may be formed. In the clustering process, vectors may be grouped on the basis of the similarity between the latent vectors.

Assuming that there are four clusters of a first cluster, a second cluster, a third cluster, and a fourth cluster, these clusters may represent different electrocardiogram patterns respectively. For example, the first cluster may include signals having normal heartbeat patterns, and the second cluster may include signals having specific abnormalities such as arrhythmia. The third cluster and the fourth cluster may include signals reflecting different types of heart diseases or conditions, respectively. In the above description, it may be explained that four clusters (or groups) may be formed, but this is only an example, and the actual number of clusters may vary depending on the characteristics of the data and the settings of the clustering model.

Such clustering may automatically identify specific patterns or abnormalities in electrocardiogram data and provide useful information for early diagnosis and management of heart disease by analyzing the features of each cluster. The clustering results may provide important data for medical professionals to comprehensively evaluate a patient's condition and establish a customized treatment plan.

In addition, the computing device 100 may build a vector database on the basis of latent vectors and information on each cluster corresponding to each latent vector in the step S3400.

The vector database of the present disclosure may be a NoSQL database server that stores and manages embedding vectors, information on the belonging cluster, and information on the distance between each vector.

The vector database of the present disclosure may be optimized for the analysis and management of latent vectors extracted from various electrocardiogram data. Through this, characteristic patterns of electrocardiogram signals may be systematically stored, retrieved and utilized when necessary.

For example, the vector database of the present disclosure may support risk evaluation and condition monitoring on the basis of a patient's electrocardiogram data. When receiving a newly collected electrocardiogram data of a specific patient, the similarity may be evaluated by comparing the latent vector extracted from the corresponding data with existing vectors stored in the vector database. Through this similarity evaluation, it may be possible to identify which cluster the electrocardiogram data of the patient belongs to and which patterns previously observed the corresponding cluster is similar to.

In addition, in an exemplary embodiment, the computing device 100 may extract a representative vector, that is, a principal vector, for each of the plurality of clusters corresponding to the clustering result. Such a principal vector may be a vector representing each cluster and may represent the center of latent vectors within the cluster.

More specifically, when the data stored in the vector database is massive, for example, it may take a long time to search for 300,000 electrocardiogram data. In general, when new electrocardiogram data of a specific patient is inputted, the data may be segmented into the plurality of ROI signals in the preprocessing process, and 80 to 120 embedding vectors may be generated through the embedding process for each ROI signal. For example, when new electrocardiogram data is individually compared with all vectors stored in the database, it may be inefficient due to the large amount of computation. This may be time-consuming and may be not suitable for situations where real-time analysis is required.

Accordingly, in order to shorten the search time and increase the efficiency, a similar cluster may be quickly identified by comparing latent vectors generated on the basis of a patient's electrocardiogram data with the principal vector of each cluster. In this process, the similarity may be evaluated by calculating the inner product between each embedding vector and the principal vector and on the basis of this information it may be determined to which cluster the corresponding electrocardiogram data belongs.

Instead of comparing all data one by one, this method may allow to quickly identify the relevant clusters by comparing with the principal vector representing each cluster. As a result, it may enable quick analysis and efficient management of the electrocardiogram data and may play an important role in rapidly evaluating the risk of heart disease and monitoring a patient's condition in real time.

In addition, in an exemplary embodiment, information on heart disease prediction for the electrocardiogram data of a subject to be predicted may be generated through the following process.

In an exemplary embodiment, the method of generating information on heart disease prediction for the electrocardiogram data of a subject to be predicted may include obtaining the electrocardiogram data of the subject to be predicted, performing preprocessing on the electrocardiogram data of the subject to be predicted, generating a plurality of latent vectors corresponding to the electrocardiogram data in which preprocessing is completed by utilizing an embedding model, classifying each of the plurality of latent vectors into one of the plurality of clusters by comparing each of the plurality of latent vectors with each representative vector corresponding to each of the plurality of clusters, and generating stratified information on the risk of heart disease on the basis of the classification result in which each of the plurality of latent vectors is classified into the plurality of clusters.

In an exemplary embodiment, the subject to be predicted may be an individual who has undergone an electrocardiogram examination for the purpose of evaluating and managing the risk of heart disease. These may be people undergoing general health screenings, people with a family history of heart disease, or patients who have been previously diagnosed with heart disease or have symptoms related to heart disease. The subject to be predicted may utilize the computing device 100 of the present disclosure to continuously evaluate the heart condition through regular electrocardiogram monitoring and to enable early detection and prevention of heart disease.

To explain in more detail, after obtaining the electrocardiogram data of the subject to be predicted, the data may be preprocessed to remove noise and refine the signal. Herein, the preprocessing may include a process of removing unnecessary signal components and emphasizing important signal features in order to improve the quality of electrocardiogram data as described above, and may include a process of segmenting and processing each electrocardiogram signal into appropriate units (a predefined window size). The predefined window size may be generally defined as a section containing important information in the electrocardiogram data, for example, as a length of about 30 seconds to 1 minute. In this process, the electrocardiogram data may be segmented at regular time intervals, each of which can be analyzed independently.

The preprocessed electrocardiogram data may be inputted to an embedding model (e.g., an encoder extracted from the self-reconstruction model pre-trained through the first learning data set), and a latent vector (or an embedding vector) may be generated from each data. The generated latent vectors may be compared with existing vectors stored in the vector database, and each vector may be classified into a specific cluster according to the similarity. Since each cluster has a specific pattern reflecting the heart condition, the risk of heart disease may be evaluated according to which cluster the latent vector belongs to.

In addition, the computing device 100 may generate stratified information on the risk of heart disease on the basis of the classification result in which the plurality of latent vectors are classified into the plurality of clusters. More specifically, in the case of electrocardiogram data of the subject to be predicted, it may be obtained as the plurality of ROI signals after being segmented in a specific time unit in the preprocessing process, and the latent vectors for each of the plurality of ROI signals may be generated as each ROI signal is processed as an input of the embedding model. In this case, information on predicting the risk of heart disease may be generated according to the classification result regarding how much each latent vector is classified into which cluster.

For a more specific example, when the electrocardiogram data of the subject to be predicted is configured with 10 ROI signals of 30 seconds, each of the corresponding 10 ROI signals may be transformed into 10 latent vectors through the embedding model, and the corresponding 10 vectors may be classified into one of four predefined clusters. Herein, each predefined cluster may represent a specific heart condition, a first cluster may be a cluster representing the highest risk, and it may be a cluster having a lower risk toward the fourth cluster.

The computing device 100 may predict the overall risk according to how the 10 input signals are classified into each cluster.

For example, when eight signals belong to the first cluster and two signals belong to the second cluster, the patient's risk of heart disease may be evaluated as very high. On the other hand, when five signals belong to the second cluster, three signals to the third cluster, and two signals to the fourth cluster, the risk may be evaluated as moderate. When two signals belong to the third cluster and eight signals to the fourth cluster, the risk may be evaluated as low, and when all ten signals belong to the fourth cluster, the patient's risk of heart disease may be evaluated as very low.

That is, the computing device 100 of the present disclosure may obtain latent vectors by analyzing ROI signals of electrocardiogram data by utilizing the embedding model, and may be capable of predicting the patient's risk of heart disease in a stratified manner by evaluating which cluster among predefined clusters each latent vector belongs to through the clustering model on the basis of the learning data set. In other words, the computing device 100 may compare latent vectors generated through the embedding model with predefined clusters to identify the cluster to which each vector belongs, and may multidimensionally evaluate the patient's heart condition using the information.

The method of predicting the risk in a stratified manner may increase the precision of electrocardiogram data analysis and help to provide a comprehensive understanding of the patient's heart condition. Accordingly, the computing device of the present disclosure may provide important information for medical professionals to establish appropriate preventive measures or treatment plans by systematically and clearly stratifying and providing a patient's risk of heart disease. Through this, this may enable early detection and appropriate response to heart disease, thereby contributing to the health care of patients.

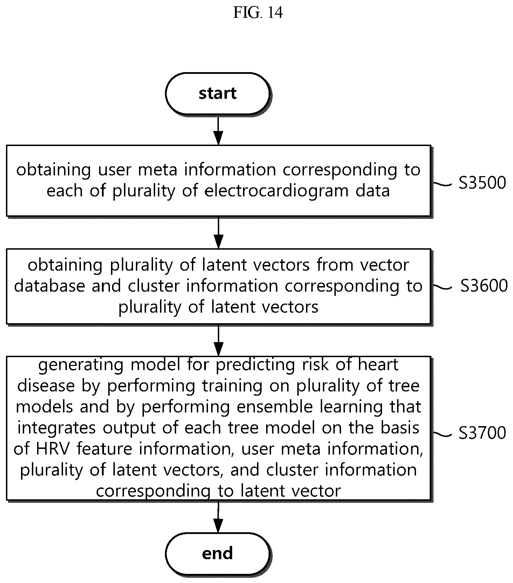

FIG. 14 is a view showing an exemplary flowchart for explaining a process of generating a model for predicting the risk of heart disease through ensemble learning of a plurality of tree-based models according to an exemplary embodiment of the present disclosure.

Referring to FIG. 14, the generating of the model for predicting the risk of heart disease may include obtaining user meta information corresponding to each of the plurality of electrocardiogram data S3500, obtaining a plurality of latent vectors and cluster information corresponding to the plurality of latent vectors from a vector database S3600, and generating the model for predicting the risk of heart disease by performing training on the plurality of tree models and performing ensemble learning that integrates the outputs of each tree model on the basis of the HRV feature information, the user meta information, the plurality of latent vectors, and the cluster information corresponding to the latent vectors S3700.

More specifically, in the step S3500, the obtaining the user meta information may include a process of collecting personal health information such as the age, gender, medical history, lifestyle, etc. of a patient. Such information may reflect well the health conditions and characteristics of the individual and may have an important impact on the interpretation of electrocardiogram data. In particular, such meta information may be utilized as an important variable necessary to more accurately evaluate the risk of heart disease. Through this, customized prediction and evaluation may be possible in consideration of the patient's personal health factors.

In the step S3600, the plurality of latent vectors and cluster information corresponding to each vector may be obtained from the vector database. The latent vector may summarize the features of the electrocardiogram data, and the cluster information may represent a pattern of a specific heart condition or disease to which the corresponding vector belongs. Through this, it may be possible to identify which cluster the patient's electrocardiogram data belongs to and to evaluate the risk of heart disease represented by the cluster.

In the step S3700, the plurality of tree models may be trained on the basis of the HRV feature information, user meta information, latent vectors, and cluster information. Each tree model may predict the risk of heart disease by using various tree-based machine learning algorithms such as Decision Tree, Random Forest, and Gradient Boosting Tree.

Herein, the cluster information may be obtained from a vector database, and may include the cluster information to which each vector belongs and the distance information between the vectors. For example, the cluster information may provide information on which cluster each latent vector belongs to and distance information representing the similarity or difference between vectors within the cluster. Such information may play an important role in evaluating which cluster a specific electrocardiogram pattern belongs to and how similar or different this pattern is compared to other electrocardiogram data. Through this, it may be possible to analyze features of electrocardiogram data and more accurately identify whether the corresponding data is related to a specific disease or condition.

More specifically, a process of training the plurality of tree-based models on the basis of the HRV feature information, user meta information, latent vectors, and cluster information may be as follows.

First, the HRV feature information may be an indicator of heart rate variability extracted from electrocardiogram data and may reflect the activity of the cardiac autonomic nervous system. The user meta information may be information representing the personal health status, such as a patient's age, gender, medical history, lifestyle, or the like. The latent vectors may be values representing features of the electrocardiogram data in a low-dimensional space, and the cluster information may represent which heart condition or disease these vectors are associated with.

These various pieces of information may be used as input variables to train the plurality of tree-based models. For example, a random forest model may construct several decision trees by using these input variables and may provide a final prediction by synthesizing the results of each tree. The gradient boosting tree may correct errors in the previous model at each stage through successive model training and may aim at more precise prediction.

In this case, the cluster information may be particularly important. The cluster to which each latent vector belongs and the distance information from other vectors may be used to further understand the features of electrocardiogram data and to evaluate the risk of heart disease. Such information may act as an important decision factor in the plurality of tree models, and each model may learn the patterns of the data on the basis of this information.

In the final ensemble process, a single comprehensive prediction result may be derived by integrating results of discrete tree models. In this process, the reliability and accuracy of the final prediction may be increased by adjusting the weight of each model. The ensemble process may provide more robust and consistent predictions by complementing the weaknesses of discrete models and combining strengths.

Consequently, this comprehensive training and ensemble process may enable to accurately and precisely predict the risk of heart disease by reflecting the multidimensional nature of electrocardiogram data.

Subsequently, ensemble learning that integrates the outputs of each tree model may be performed, which increases the accuracy and reliability of the final prediction by combining the prediction results of the discrete models. Through ensemble learning, the risk of heart disease may be predicted more precisely, thereby comprehensively evaluating the patient's health condition and establishing an early diagnosis and preventive treatment plan.

That is, the present disclosure may provide higher prediction performance than a single model by combining the strengths of various models through ensemble learning for the plurality of tree-based models and improve the robustness against noise or fluctuations in the data. Through this, the model may provide consistent predictions for various patient data, and may reduce the uncertainty of predictions.

In summary, the computing device of the present disclosure may utilize three neural network models in order to more accurately predict the risk of heart disease. First, the embedding model may summarize important signal features by transforming electrocardiogram data into low-dimensional latent vectors. Second, the clustering model may determine which cluster each vector belongs to by clustering latent vectors. Finally, the model of predicting the risk of heart disease may predict the risk of heart disease by combining the HRV feature information, user meta information, latent vectors, and cluster information. Through this integrated approach method, the computing device may predict the risk of heart disease more precisely and consistently.

In particular, the plurality of tree-based models may be used in the final process of predicting the risk of heart disease by utilizing four variables such as the HRV feature information, user meta information, latent vectors, and cluster information and these models may generate each output. The final output, that is, the result of predicting the risk of heart disease may be generated by integrating the outputs through ensemble learning. Through this configuration, the accuracy of predicting the risk of heart disease may be improved, and a more multidimensional and comprehensive evaluation may be possible through a combination of various variables. This may reflect the patient's individual health condition more accurately and may play an important role in helping medical staff clearly understand the patient's risk and establish the optimal treatment plan.

Accordingly, the model of predicting the risk of heart disease generated through the final training completion may stratify and provide prediction information on the risk of future heart disease on the basis of the patient's electrocardiogram data. The stratified information may play an important role in multi-dimensionally evaluating the patient's heart health condition and establishing individual treatment plans. This may contribute to the early detection and management of heart disease by allowing medical staff to more clearly understand the patient's risk and take more precise treatment and preventive measures.

Specifically, this system may comprehensively analyze the electrocardiogram data and patient's meta-information to classify the possibility of developing heart disease into various risk levels. For example, it may be possible for medical staff to clearly understand how much attention they should pay to a specific patient by providing risk levels stratified into low, medium, and high. By this, high-risk patients may be more likely to receive early diagnosis and treatment, and low-risk patients may reduce unnecessary examinations and receive efficient health management. As a result, the system may play an important role in supporting the efficient allocation of healthcare resources, preventing complications from heart disease, and improving patients' quality of life.

The exemplary embodiments described above may be implemented by hardware components, software components, and/or a combination of hardware components and software components. For example, the devices, methods, and components described in the exemplary embodiments may be implemented using a general purpose computer or a special purpose computer, such as, for example, a processor, controller, arithmetic logic unit (ALU), digital signal processor, microcomputer, field programmable gate array (FPGA), programmable logic unit (PLU), microprocessor, or any other device capable of executing and responding to instructions. The processing device may perform an operating system (OS) and a software application executed on the operating system. In addition, the processing device may access, store, manipulate, process, and generate data in response to the execution of software. For ease of understanding, the processing device may be sometimes described as being used alone, but those skilled in the art will recognize that the processing device may include a plurality of processing elements and/or a plurality of types of processing elements. For example, the processing device may include a plurality of processors or one processor and one controller. In addition, other processing configurations, such as parallel processors, may be possible as well.

The software may include a computer program, code, instruction, or a combination of one or more thereof, and may configure the processing device to operate as desired or may independently or collectively command the processing device. Software and/or data may be stored in any type of machine, component, physical device, virtual equipment, computer storage medium or device in order to be interpreted by the processing device or to provide instructions or data to the processing device. Software may be distributed over a networked computer system, and stored or executed in a distributed manner. Software and data may be stored in a computer-readable recording medium.

The methods according to an exemplary embodiment may be implemented in the form of program instructions that may be executed through various computer means and may be recorded on a computer-readable medium. A computer-readable medium may store program instructions, data files, data structures, and the like alone or in combination, and program instructions recorded on the medium may be specially designed and configured for exemplary embodiments or may be known and available to those skilled in the computer software art. Examples of computer-readable recording media may include magnetic media such as hard disks, floppy disks and magnetic tapes, optical media such as CD-ROMs and DVDs, magnetic-optical media such as floptical disks, and hardware devices specifically configured to store and perform program instructions such as ROMs, RAMs, flash memories, and the like. Examples of program instructions may include not only machine language codes, such as that made by a compiler, but also high-level language codes that may be executed by a computer using an interpreter or the like.

The hardware device described above may be configured to operate as one or more software modules in order to perform the operations of the exemplary embodiments, and vice versa.

The exemplary embodiments have been described with limited drawings as described above, but those skilled in the art may apply various technical modifications and variations based on that. For example, appropriate results can be achieved even when the described techniques are performed in an order different from the described method, and/or described components such as systems, structures, devices, circuits, etc. are combined or joined in a form different from the described method, or substituted or replaced by other components or equivalents.

Therefore, other implementations, other exemplary embodiments, and those equivalent to the claims may fall within the scope of the claims described below.

**Claims**

1. A method for training a deep learning model for predicting heart disease, which is performed by a processor included in a computing device, the method comprising:

   obtaining, by the processor, discontinuous discrete heartbeats from electrocardiogram data, which are unstructured data obtained from a plurality of patients as well as consisting of continuous signals; and
   training, by the processor, the deep learning model so as to predict each heart disease class of the obtained discrete heartbeats by inputting the obtained discrete heartbeats into the deep learning model for predicting the patient's heart disease.

2. The method of claim 1, wherein the training comprises:
   training the deep learning model by using a first discrete heartbeat obtained from at least one piece of electrocardiogram data among the obtained discrete heartbeats.

3. The method of claim 1, wherein the obtaining comprises:

   removing noise by filtering each of the electrocardiogram data; and
   segmenting each of the electrocardiogram data into the discrete heartbeats by applying a peak detection algorithm to each of the electrocardiogram data with the noise removed.

4. The method of claim 1, wherein the training comprises:
   optimizing a parameter of the deep learning model by using a binary cross entropy and an optimizer.

5. The method of claim 1, further comprising:
   determining, by the processor, a threshold value for distinguishing the heart disease class by applying second discrete heartbeats obtained from at least one piece of electrocardiogram data among the obtained discrete heartbeats to the trained deep learning model.

6. The method of claim 5, wherein the determining comprises:

   collecting a probability score for each of the discrete heartbeats separated from the same electrocardiogram data for each of the second discrete heartbeats;
   deriving a final probability score by averaging the probability score for each of the collected second discrete heartbeats; and
   fine-tuning the threshold value for distinguishing the heart disease class on the basis of the derived final probability score.

7. The method of claim 5, wherein the determined threshold value is stored together with a weight of the deep learning model.

8. A method for predicting heart disease by using a deep learning model, which is performed by a processor included in a computing device, the method comprising:

   receiving, by the processor, electrocardiogram data, which are unstructured data consisting of continuous signals with respect to a patient;
   obtaining discontinuous discrete heartbeats from the received electrocardiogram data;
   deriving a probability value to be predicted as a specific heart disease class for each of the obtained discrete heartbeats by inputting the obtained discrete heartbeats into the deep learning model; and
   predicting the patient's heart disease by using the derived probability values,

wherein the deep learning model is a model trained using the discrete heartbeats obtained from the electrocardiogram data of the plurality of patients.

9. The method of claim 8, wherein the predicting comprises:

distinguishing the patient's heart disease class by comparing an average of the derived probability values with a threshold value,
wherein the threshold value is determined by applying the discrete heartbeats obtained from at least one piece of electrocardiogram data among the electrocardiogram data of the plurality of patents to the deep learning model and is a value for distinguishing the heart disease class.

10. The method of claim 8, wherein the obtaining comprises:

removing a noise by filtering the received electrocardiogram data; and
segmenting the electrocardiogram data into the discrete heartbeats by applying a peak detection algorithm to the electrocardiogram data with the noise removed.

11. The method of claim 8, wherein the deep learning model is in a state where a parameter is optimized on the basis of a binary cross entropy and an optimizer.

12. A computing device comprising:

a processor;
a network interface;
a memory; and
a computer program which is loaded into the memory and executed by the processor,
wherein the processor performs the method of claim 1 by executing one or more instructions included in the computer program.

13. A computing device comprising:

a processor;
a network interface;
a memory; and
a computer program which is loaded into the memory and executed by the processor,
wherein the processor performs the method of claim 8 by executing one or more instructions included in the computer program.

14. A computer program stored in a computer-readable non-transitory recording medium while being combined with a computing device in order to perform the method of claim 1.

15. A computer program stored in a computer-readable non-transitory recording medium while being combined with a computing device in order to perform the method of claim 8.

FIG. 1

100

## computing device

110

120

memory

### processor

130

### program

140

### display

FIG. 2

```
                    ┌──────────┐
                    │  start   │
                    └────┬─────┘
                         │
                         ▼
┌─────────────────────────────────────────────────┐
│ classifying electrocardiogram data obtained from  │
│ plurality of patients into training data set,      │  ~S210
│ validation data set, and test data set            │
└─────────────────────┬───────────────────────────┘
                      │
                      ▼
┌─────────────────────────────────────────────────┐
│ obtaining discrete heartbeats from each of        │
│ electrocardiogram data classified into training    │  ~S220
│ data set, validation data set, and test data set   │
└─────────────────────┬───────────────────────────┘
                      │
                      ▼
┌─────────────────────────────────────────────────┐
│ training deep learning model to predict heart      │
│ disease class of each of first discrete heartbeats  │
│ in response to inputting first discrete heartbeats  │
│ obtained from electrocardiogram data included in    │  ~S230
│ training data set into deep learning model for      │
│ predicting patient's heart disease                  │
└─────────────────────┬───────────────────────────┘
                      │
                      ▼
┌─────────────────────────────────────────────────┐
│ determining threshold value for distinguishing     │
│ heart disease class by applying second discrete     │
│ heartbeats obtained from electrocardiogram data     │  ~S240
│ included in validation data set to trained deep     │
│ learning model                                      │
└─────────────────────┬───────────────────────────┘
                      │
                      ▼
                 ┌──────────┐
                 │   end    │
                 └──────────┘
```

FIG. 3

310 electrocardiogram data (T-NSR, AF-NSR, CIA-NSR)

320 electrocardiogram data (T-NSR, AF-NSR)

330 electrocardiogram data (T-NSR, CIA-NSR)

**AF-NSR branch:**

training data set(60%)
AF-NSR ECGs : 3,225
T-NSR ECGs : 21,028
2017-05-23~2021-06-10

validation data set(20%)
AF-NSR ECGs : 1,073
T-NSR ECGs : 6,972
2017-05-23~2021-06-10

test data set(20%)
AF-NSR ECGs : 1,385
T-NSR ECGs : 6,514
2021-06-11~2022-05-23

**CIA-NSR branch:**

training data set(60%)
CIA-NSR ECGs : 6,855
T-NSR ECGs : 21,028
2017-05-23~2021-06-10

validation data set(20%)
CIA-NSR ECGs : 2,329
T-NSR ECGs : 6,972
2017-05-23~2021-06-10

test data set(20%)
CIA-NSR ECGs : 2,543
T-NSR ECGs : 6,514
2021-06-11~2022-05-23

**AF-NSR HB:**

training data set(60%)
AF-NSR HB : 604,700
T-NSR HB : 3,177,263
2017-05-23~2021-06-10

validation data set(20%)
AF-NSR HB : 198,672
T-NSR HB : 1,058,107
2017-05-23~2021-06-10

test data set(20%)
AF-NSR HB : 228,708
T-NSR HB : 977,875
2021-06-11~2022-05-23

**CIA-NSR HB:**

training data set(60%)
CIA-NSR HB : 1,113,089
T-NSR HB : 3,177,263
2017-05-23~2021-06-10

validation data set(20%)
CIA-NSR HB : 375,700
T-NSR HB : 1,058,107
2017-05-23~2021-06-10

test data set(20%)
CIA-NSR HB : 423,581
T-NSR HB : 977,875
2021-06-11~2022-05-23

FIG. 4

FIG. 5a

Resnet-18 architecture

Conv 1x15 (64) | Bath norm | Activation
[B, 700]
[B, 5000, 12]

Resblock 1: Conv BN ReLU Conv BN

Resblock 4: Conv BN ReLU Conv BN

Average pool | Fully connected

Output : [B, 2]

Feature extraction

Conv 1x3 (128) | ReLU | Conv 1x3 (64) | ReLU

Conv 1x3 (64) | ReLU | Max pool | Conv 1x3 (64) | ReLU | Max pool

[B, 700]
[B, 5000, 12]

Transformer encoder & decoder

Positional Encoder | Max pool | Self-attention pooling | Tranformer decoder | Flatten

Fully connected

Output : [B, 2]

Conv1D + LSTM Architecture

FIG. 5b

FIG. 5c

[B, 700]
[B, 5000, 12]

Output : [B, 2]

Conv1D + Transformer architecture

FIG. 6

FIG. 7

```
                        ┌──────────┐
                        │  start   │
                        └──────────┘
                             │
                             ▼
┌──────────────────────────────────────────────────────────┐
│ receiving electrocardiogram data of patient whose heart   │──S710
│           disease class is to be predicted                │
└──────────────────────────────────────────────────────────┘
                             │
                             ▼
┌──────────────────────────────────────────────────────────┐
│        obtaining discrete heartbeats from received        │──S720
│                  electrocardiogram data                   │
└──────────────────────────────────────────────────────────┘
                             │
                             ▼
┌──────────────────────────────────────────────────────────┐
│ inputting obtained discrete heartbeats into deep learning │
│ model for predicting patient's heart disease and deriving │──S730
│ probability value to be predicted as specific heart disease│
│           class for each of discrete heartbeats           │
└──────────────────────────────────────────────────────────┘
                             │
                             ▼
┌──────────────────────────────────────────────────────────┐
│   predicting patient's heart disease by using probability │──S740
│        values derived for each of discrete heartbeats     │
└──────────────────────────────────────────────────────────┘
                             │
                             ▼
                        ┌──────────┐
                        │   end    │
                        └──────────┘
```

FIG. 8

FIG. 9

```
            ┌─────────┐
            │  start  │
            └────┬────┘
                 │
                 ▼
  ┌─────────────────────────────────────────┐
  │  obtaining plurality of electrocardiogram│ ─── S1000
  │                data                      │
  └─────────────────┬───────────────────────┘
                    │
                    ▼
  ┌─────────────────────────────────────────┐
  │ constructing learning data set based on  │ ─── S2000
  │    plurality of electrocardiogram data   │
  └─────────────────┬───────────────────────┘
                    │
                    ▼
  ┌─────────────────────────────────────────┐
  │ generating model for predicting risk of  │
  │   heart disease by performing training   │ ─── S3000
  │  on one or more network functions on the │
  │       basis of learning data set         │
  └─────────────────┬───────────────────────┘
                    │
                    ▼
            ┌─────────┐
            │   end   │
            └─────────┘
```

FIG. 10

```
                    ╭─────────────╮
                    │    start    │
                    ╰──────┬──────╯
                           │
                           ▼
    ┌──────────────────────────────────────────┐
    │   performing noisy preprocessing on        │
    │         plurality of                        │  ⌐ S2100
    │      electrocardiogram data                 │
    └──────────────────────┬─────────────────────┘
                           │
                           ▼
    ┌──────────────────────────────────────────┐
    │  obtaining plurality of ROI signals by     │
    │          segmenting                         │
    │  plurality of noisy preprocessed            │  ⌐ S2200
    │  electrocardiogram data                     │
    │     into predefined window size             │
    └──────────────────────┬─────────────────────┘
                           │
                           ▼
    ┌──────────────────────────────────────────┐
    │  extracting HRV feature information in      │
    │         lead unit of                        │  ⌐ S2300
    │   plurality of electrocardiogram data       │
    └──────────────────────┬─────────────────────┘
                           │
                           ▼
                    ╭─────────────╮
                    │     end     │
                    ╰─────────────╯
```

FIG. 11

```
start
```

| generating embedding model through masking-based self-directed learning utilizing first learning data set | ~S3100 |

| performing embedding that extracts latent vectors corresponding to each of plurality of ROI signals corresponding to second learning data set by utilizing embedding model | ~S3200 |

| performing clustering on latent vectors by utilizing clustering model | ~S3300 |

| constructing vector database on the basis of latent vectors and information on each cluster corresponding to each of latent vectors | ~S3400 |

```
end
```

FIG. 12

```
                         ┌─────────────┐
                         │    start    │
                         └──────┬──────┘
                                │
                                ▼
    ┌──────────────────────────────────────────────┐
    │  deriving self-supervised learning that allows self-│
    │  reconstruction model to generate output similar to │
    │  inputted data by processing data included in first │──S3110
    │  learning data set as input for self-reconstruction │
    │                    model                       │
    └──────────────────────┬───────────────────────┘
                           │
                           ▼
    ┌──────────────────────────────────────────────┐
    │  generating embedding model by extracting encoder│
    │  from self-reconstruction model for which training is│──S3120
    │                  completed                     │
    └──────────────────────┬───────────────────────┘
                           │
                           ▼
                    ┌─────────────┐
                    │     end     │
                    └─────────────┘
```

FIG. 13

FIG. 14

```
                    ┌─────────────┐
                    │    start    │
                    └─────────────┘
                           │
                           ▼
    ┌──────────────────────────────────────────────┐
    │  obtaining user meta information corresponding to  │ ── S3500
    │     each of plurality of electrocardiogram data    │
    └──────────────────────────────────────────────┘
                           │
                           ▼
    ┌──────────────────────────────────────────────┐
    │   obtaining plurality of latent vectors from vector  │
    │  database and cluster information corresponding to  │ ── S3600
    │            plurality of latent vectors              │
    └──────────────────────────────────────────────┘
                           │
                           ▼
    ┌──────────────────────────────────────────────┐
    │   generating model for predicting risk of heart     │
    │  disease by performing training on plurality of tree │
    │  models and by performing ensemble learning that    │
    │  integrates output of each tree model on the basis   │ ── S3700
    │  of HRV feature information, user meta information,   │
    │  plurality of latent vectors, and cluster information │
    │         corresponding to latent vector              │
    └──────────────────────────────────────────────┘
                           │
                           ▼
                    ┌─────────────┐
                    │     end     │
                    └─────────────┘
```

Europäisches
Patentamt

European
Patent Office

Office européen
des brevets

## EUROPEAN SEARCH REPORT

**Application Number**

EP 25 19 5232

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZEKAI WANG ET AL: "Hierarchical Deep Learning with Generative Adversarial Network for Automatic Cardiac Diagnosis from ECG Signals", ARXIV.ORG, CORNELL UNIVERSITY LIBRARY, 201 OLIN LIBRARY CORNELL UNIVERSITY ITHACA, NY 14853, 19 October 2022 (2022-10-19), XP091349268, * section 3 * * section 3.1.3 * * section 3.2 * * section 4.1 * * section 4.2 * ----- | 1-15 | INV. A61B5/024 A61B5/0245 A61B5/352 A61B5/361 A61B5/00 G06N3/045 G16H50/20 G16H50/30 G16H50/70 G06N3/044 G06N3/0455 G06N3/0464 G06N3/047 |
| X | ISLAM MD SHOFIQUL ET AL: "HARDC : A novel ECG-based heartbeat classification method to detect arrhythmia using hierarchical attention based dual structured RNN with dilated CNN", NEURAL NETWORKS, ELSEVIER SCIENCE PUBLISHERS, BARKING, GB, vol. 162, 5 March 2023 (2023-03-05), pages 271-287, XP087301051, ISSN: 0893-6080, DOI: 10.1016/J.NEUNET.2023.03.004 [retrieved on 2023-03-05] * section 1 * * section 3 * * section 3.2 * * section 4.1 * * section 4.3.1 * * section A.1.1 * ----- -/-- | 1-15 | G06N3/0475 G06N3/048 G06N3/08 G06N3/084 |

| TECHNICAL FIELDS SEARCHED (IPC) |
|---|
| A61B G06N G16H |

The present search report has been drawn up for all claims

| Place of search | Date of completion of the search | Examiner |
|---|---|---|
| The Hague | 27 October 2025 | Gooding Arango, J |

EPO FORM 1503 03.82 (P04C01)

page 1 of 2

Europäisches Patentamt
European Patent Office
Office européen des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/245782 A1 (ZIMMERMAN NOAH [US] ET AL) 3 August 2023 (2023-08-03)<br>* paragraph [0137] *<br>* paragraph [0146] - paragraph [0159] *<br>* paragraph [0202] - paragraph [0215] *<br>* paragraph [0232] *<br>* paragraph [0295] *<br>* paragraph [0347] *<br>* paragraph [0375] - paragraph [0380] *<br>* figure 5A *<br>* table 4 *<br>----- | 1-15 | G06N3/088<br>G06N3/09<br>G06N3/094<br>G06N5/01<br>G06N7/01<br>G06N20/00<br>G06N20/10<br>G06N20/20<br>G16H50/50 |
| A | US 2022/095982 A1 (DE SAINT VICTOR MARIE-ALBANE [FR] ET AL) 31 March 2022 (2022-03-31)<br>* paragraph [0019] - paragraph [0027] *<br>* paragraph [0086] *<br>* paragraph [0101] *<br>* paragraph [0119] - paragraph [0124] *<br>* paragraph [0129] *<br>* paragraph [0151] *<br>----- | 1-15 | |

TECHNICAL FIELDS
SEARCHED (IPC)

The present search report has been drawn up for all claims

| Place of search | Date of completion of the search | Examiner |
|---|---|---|
| The Hague | 27 October 2025 | Gooding Arango, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

## ANNEX TO THE EUROPEAN SEARCH REPORT
## ON EUROPEAN PATENT APPLICATION NO.

EP 25 19 5232

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2025

| Patent document cited in search report | Publication date | Patent family member(s) | Publication date |
|---|---|---|---|
| US 2023245782 A1 | 03-08-2023 | US 2022378379 A1<br>US 2023245782 A1 | 01-12-2022<br>03-08-2023 |
| US 2022095982 A1 | 31-03-2022 | AU 2021351233 A1<br>CA 3197070 A1<br>CN 116234497 A<br>EP 4221579 A1<br>JP 2023543836 A<br>US 2022095982 A1<br>WO 2022070109 A1 | 08-06-2023<br>07-04-2022<br>06-06-2023<br>09-08-2023<br>18-10-2023<br>31-03-2022<br>07-04-2022 |

EPO FORM P0459

For more details about this annex : see Official Journal of the European Patent Office, No. 12/82

**EP 4 661 028 A1**

**Patent documents cited in the description**

- KR 1020230110112
- KR 1020240104439
- KR 1020220104583